# EUROPÄISCHE PATENTSCHRIFT

(11) **EP 0 369 333 B1**
(45) Veröffentlichungstag und Bekanntmachung des Hinweises auf die Patenterteilung: **08.03.1995**
(21) Anmeldenummer: 89120827.4
(22) Anmeldetag: 10.11.1989
(51) Int. Cl.: C12Q 1/56

(54) **Globaltest zur Erfassung der Hauptkomponenten des Fibrinolysesystems**
Universal test for the main components of the fibrinolytic system
Test universel pour les composants principaux du système fibrinolytique

(30) Priorität: 14.11.1988 DE 3838529
(43) Veröffentlichungstag der Anmeldung: 23.05.1990
(73) Patentinhaber: BEHRINGWERKE Aktiengesellschaft, 35001 Marburg (DE)
(72) Erfinder: Stief, Thomas, Dr., E-41007 Sevilla (ES)

(56) Entgegenhaltungen:
- EP-A- 0 173 916
- EP-A- 0 189 910
- EP-A- 0 198 322
- EP-A- 0 297 597
- JP-A- 6 336 782

## Beschreibung

Die Erfindung betrifft ein Verfahren zur globalen Erfassung der Komponenten des Fibrinolysesystems in Plasma oder anderen biologischen Flüssigkeiten, wobei ein Plasminogenaktivator zugesetzt oder in der Probe vorhandener Plasminogenaktivator aktiviert und die entstehende Plasminaktivität bestimmt wird.

Der menschliche Organismus besitzt ein enzymatisches System, welches in der Lage ist, entstandene Blutgerinnsel wieder aufzulösen: das fibrinolytische System. Während das Gerinnungssystem durch Globalteste wie die partielle Thromboplastinzeit oder die aktivierte partielle Thromboplastinzeit in seiner Funktion seit vielen Jahren einfach zu untersuchen ist, gab es auf seiten der Fibrinolyse kein derartiges Meßverfahren, welches in umfassender Weise die entscheidenden Komponenten des Systems funktionell erfaßte.

In der EP-A 0 189 910 wird ein Verfahren zur Bestimmung des fibrinolytischen Zustandes von Plasma beschrieben, bei dem man einer nativen Plasmaprobe eine zur Trübungsbildung ausreichende Menge an Fibrin zusetzt oder diese in situ erzeugt und die Trübung oder die gebildeten Fibrinspaltprodukte mißt oder ein chromogenes Plasminsubstrat und eine nicht zur Trübungsbildung ausreichende Menge an Fibrin, Fibrinogen-Spaltprodukten oder eines Fibrin in situ erzeugenden Enzyms zusetzt und die gebildete Farbe mißt.

Die wesentlichen Komponenten des fibrinolytischen Systems sind Plasminogen, alpha-2-Antiplasmin (A2-AP) und Plasminogen-Aktivator-Inhibitor (PAI), hier vor allem vom endotheliären Typ I. Alle 3 Komponenten können durch Einzelteste bestimmt werden; ein Globaltest, der das Zusammenspiel der 3 Komponenten wiedergibt, lag nach dem Stand der Technik nicht vor.

Überraschenderweise wurde gefunden, daß man durch Inkubation von Plasmaproben mit einem Plasminogen-Aktivator gemeinsam mit einer omega-Aminocarbonsäure und/oder einem methioninspezifischen Oxidans innerhalb weniger Minuten Plasminaktivität in Abhängigkeit von Plasminogen, alpha-2-Antiplasmin und Plasminogen-Aktivator-Inhibitor erzeugen und somit das fibrinolytische System eines Patienten untersuchen kann, indem die entstandene Plasminaktivität z. B. über ein chromogenes oder fluorogenes Plasminsubstrat bestimmt wird. Aus der gemessenen Plasminaktivität wird durch Vergleich mit den Plasminaktivitäten von Normalplasma die globale Funktion der Komponenten des Fibrinolysesystems in Plasma festgestellt. Verwendet man als Aktivator t-PA und setzt im Testansatz einen Chelatbildner wie EDTA in einer Konzentration von 0.5 - 10 mM ein, so erfaßt der Test auch die Stimulation von t-PA durch Fibrin, eine weitere wichtige Größe der Fibrinolyse.

Wird statt Plasminogen-Aktivator ein Kontaktphasen-Aktivator wie Ellagsäure (1-100 »g/Ansatz) oder ein Sulfatid verwendet, mißt der Test den Intrinsic-Weg der Fibrinolyse über plasmatische Prourokinase, Faktor XIIa und Kallikrein, benötigt allerdings um den Faktor 5 längere Inkubationszeiten.

Gegenstand der Erfindung ist daher ein Verfahren zur globalen Erfassung der Komponenten des Fibrinolysesystems im Plasma oder anderen biologischen Flüssigkeiten dadurch gekennzeichnet, daß man Plasma oder dieser Flüssigkeit einen Plasminogenaktivator, eine ω̅-Aminokarbonsäure und/oder ein Methionin zu Methioninsulfoxid oxidierendes Agens zusetzt und die entstehende Plasminogenaktivität bestimmt, wonach durch Vergleich mit der Plasminogenaktivität von Normalplasma die globale Funktion der Komponenten des Fibrinolysesystems in Plasma festgestellt wird.

Es wird soviel Plasminogenaktivator zugesetzt, daß die Konzentration von t-PA 2 - 200 IE/ml oder die von Urokinase 1-100 IE/ml beträgt.

Zur Durchführung des erfindungsgemäßen Verfahrens wird zu 50 - 200 »l (vorzugsweise 100 »l) Plasma, vorzugsweise Citratplasma, 200 »l eines Plasminogen-Aktivator-Reagenzes zugegeben, das 2.5 - 40, vorzugsweise 5 - 20 IE/ml Urokinase oder 12.5 - 100 IE/ml Gewebeplasminogen-Aktivator und gegebenenfalls zur Verbesserung der Aktivität 100 - 2000 »g/ml einer t-PA-Aktivität stimulierenden Substanz enthält.

Diese stimulierende Substanz können Fibrinogenspaltprodukte sein, die unter Ca²⁺-Entzug durch Proteolyse mit Plasmin aus Fibrin hergestellt und gelöst in 20 - 200 mM Tris, 0 - 2 % Polygeline, 0.01 % ^{R}Triton X 100, pH 7.5 - pH 9.5, vorzugsweise 8.5 eingesetzt werden. Falls der Plasminogen-Aktivator Urokinase ist, kann die Lösung, die ihn enthält, eine diesen stimulierende Substanz vorzugsweise 3 mM Tranexamsäure oder aber 30 mM epsilon-Aminocapronsäure oder 100 mM Lysin oder eine andere omega-Aminocarbonsäure enthalten. Die omega-Aminocarbonsäuren könnnen auch separat dem Reaktionsansatz zugegeben werden.

Weiterhin kann dem Reaktionsansatz bevorzugt für Messungen mit t-PA, anstelle der omega-Aminocarbonsäure, oder bevorzugt für Messungen mit u-PA, in Kombination mit der omega-Aminocarbonsäure, ein methionin-spezifisches Oxidationsmittel wie Chloramin T, Chloramin B oder HOCl zugesetzt werden, um Antiplasmin und andere Serinproteasen-Inhibitoren zu zerstören, und zwar in Konzentrationen von 0.2 - 20 mM im Testansatz. Unter methionin-spezifischen Oxidantien werden Substanzen verstanden, welche vorzugsweise bei pH 7 - pH 9, besonders bei pH 8 - pH 8.8, Methionin zu Methioninsulfoxid oxidieren.

Nach einer mehrminütigen, vorzugsweise 10minütigen, Inkubation bei 37°C wird ein chromogenes Plasminsubstrat, vorzugsweise gemeinsam mit einer Kochsalzlösung (100 - 800 mM NaCl im Reaktionsansatz)und/oder CsCl-Lösung (30 - 300 mM im Reaktionsansatz) zugegeben, um eine lineare Reaktionskinetik zu erhalten, und die Geschwindigkeit der Extinktionsänderung (delta E/t) ermittelt.

Alternativ kann die gleiche Menge an chromogenem Plasminsubstrat (ohne NaCl bzw. CsCl) in der ersten Inkubation, vorzugsweise mit dem Plasminogen-Aktivator-Reagenz, zugegeben werden, woraus jedoch eine hyperbolische Reaktionskinetik resultiert. Die Reaktion wird vorzugsweise nach ca. 5 min durch Zugabe einer Säure, z.B. 100 »l 8,5 M Essigsäure beendet.

Die Erfindung ist weiterhin in den Ansprüchen definiert und im folgenden durch Beispiele erläutert.

### Beispiel 1

### Ausführung des erfindungsgemäßen Verfahrens an verschiedenen Pathoplasmen

100 »l humanes Citratplasma a) Standardhumanplasma, b) A2-AP-Mangelplasma (d.h. kein A2-AP im Plasma; immunadsorptiv hergestellt), c) 50%iges Plasminogen-Mangelplasma (d.h. Plasminogengehalt im Plasma = 50 % der Norm; immunadsorptiv hergestellt), d) PAI-reiches Plasma mit 4.2 u-PA-inhibierenden Einheiten/ml wurden mit 200 »l u-PA-Reagenz 5, 7.5 und 10 IE u-PA/ml in 150 mM Tris, 3 mM Tranexamsäure, 1 % Polygeline, 0.01 % Triton X 100, pH 8.4) 10 min bei 37°C inkubiert. Daraufhin wurde 500 »l chromogenes Substrat HD-Norvalylcyclohexylalanyl-lysyl-paranitroanilide (HD-Nva-CHA-Lys-pNA) 0.6 mM in 480 mM NaCl, 100 mM CsCl zugesetzt, die Substratreaktion nach 3 min (37°C) durch Zugabe von 200 »l 3.4 M Essigsäure beendet und die entstandene Extinktion bei 405 nm bestimmt.

**Tabelle 1**

| | Zugabe von | | |
|---|---|---|---|
| | 1 IE u-PA | 1.5 IE u-PA | 2 IE u-PA |
| | delta E/3 min (x 1000) | | |
| a) Normalplasma | 290 | 402 | 536 |
| b) A2-AP-Mangelplasma | 696 | 863 | 929 |
| c) 50% Plasminogen-Mangelplasma | 154 | 216 | 275 |
| d) PAI-reiches Plasma (4.2 U/ml) | 234 | 329 | 428 |

Man erkennt, daß verglichen mit Normalplasma, ein Antiplasmin-Mangel zu einer verstärkten Fibrinolyse führt, resultierend in erhöhten delta-E-Werten, und daß PAI-reiches und plasminogen-armes Plasma eine verminderte Fibrinolyse, ausgedrückt in erniedrigten delta-E-Werten bedingt. Der erfindungsgemäße Test gibt folglich Aufschluß über Änderungen in der Aktivität der Schlüsselkomponenten PAI, A2-AP und Plasminogen. Die höchsten Extinktionsausbeuten bei gleichzeitig guter Sensitivität gegenüber Plasmakonzentrationsschwankungen der Fibrinolysekomponenten ergeben sich bei Zusatz von 2 IU u-PA zu 100 »l Humanplasma.

### Beispiel 2

### Einfluß von pH-Wert auf den erfindungsgemäßen Test

Beispiel 1 wurde mit Normalplasma als Probe bei verschiedenen pH-Werten durchgeführt.

**Tabelle 2**

| pH 7 | pH 7.5 | pH 8 | pH 8.5 | pH 9 | pH 9.5 |
|---|---|---|---|---|---|
| delta E/3 min (x 1000) | | | | | |
| 309 | 413 | 522 | 536 | 414 | 337 |
| Bei pH 8.5 ergibt sich ein pH-Optimum. | | | | | |

### Beispiel 3

### Einfluß von verschiedenen Tranexamsäure-Konzentrationen auf den erfindungsgemäßen Test

Beispiel 1 wurde mit Normalplasma als Probe bei verschiedenen Konzentrationen an Tranexamsäure oder Chloramin T durchgeführt.

**Tabelle 3**

| | mM | delta E/min |
|---|---|---|
| Tranexamsäurekonzentr. in 300 »l Testvolumen | 0 | 74 |
| | 0.75 | 443 |
| | 1.5 | 600 |
| | 2.25 | 553 |
| | 3 | 501 |
| Chloramin T-Konzentr. in 300 »l Testvolumen | 0 | 74 |
| | 1.25 | 103 |
| | 5 | 234 |
| | 10 | 178 |

Es ergibt sich ein Optimum bei Verwendung von ca. 1.5 mM Tranexamsäure im Reaktionsansatz. Auch die Verwendung von Chloramin T führt zu verbesserten Extinktionsausbeuten mit einem Optimum bei ca. 5 mM im Testansatz.

### Beispiel 4

### Fibrinolysetest über Kontaktphasenaktivierung von endogenem (intrinsic) Plasminogenaktivator

100 »l humanes Citratplasma a) Standardhumanplasma, b) A2-AP-Mangelplasma, c) Plasma supplementiert mit 10 »g/ml Prourokinase (sc-u-PA), d) Plasma supplementiert mit 20 ng/ml Prourokinase, e) Plasma supplementiert mit 40 IE/ml Einketten-Gewebeplasminogen-Aktivator (sc-t-PA), f) Plasma supplementiert mit 80 IE/ml sc-t-PA, g) h) i) Patientenplasmen mit hyperfibrinolytischer Anamnese (Blutungen ohne Gerinnungsdefekt ) wurden mit 100 »l Reagenz I gemischt. Reagenz I bestand aus je 1 Volumenteil ^{R}Neothromtin (Behringwerke, Marburg), gelöst in 36 ml 150 mM Tris, 50 mM NaCl, 0.02 % Triton, 1 % Polygeline, 0.01 % Natriumazid, pH 8.4 und 1 Volumenteil Tranexamsäure 12 mM.

Als Nullwertabgleich wurden vor Zusatz des Reagenz I 400 »l Stoplösung (480 mM NaCl, 100 mM CsCl, 30 mM Arginin, 50 mM Tris, pH 8.4) zur Probe gegeben.

Nach 10minütiger Inkubation bei 37°C wurden 500 »l 0.6 mM Substratlösung (HD-Nva-CHA-Lys-pNA), gelöst in aqua dest/Stoplösung im Verhältnis 1:4 bzw. für den Nullwert 100 »l 3 mM Substratlösung in aqua dest. zugefügt, 60 min bei 37°C inkubiert, die Reaktion durch Zusatz von 250 »l 3.4 M Essigsäure beendet und die entstandene Extinktion bei 405 nm bestimmt.

**Tabelle 4**

| Plasma | erzeugte Plasminaktivität (E x 1000) |
|---|---|
| a) Standardhumanplasma | 284 ± 1 |
| b) A2-AP-Mangelplasma | 453 ± 0 |
| c) a) + 10 ng/ml sc-u-PA | 376 ± 1 |
| d) a) + 20 ng/ml sc-u-PA | 440 ± 5 |
| e) a) + 40 IE/ml sc-t-PA | 374 ± 5 |
| f) a) + 80 IE/ml sc-t-PA | 437 ± 3 |
| g) Patient 1 | 205 ± 10 |
| h) Patient 2 | 551 ± 17 |
| i) Patient 3 | 502 ± 6 |

Man erkennt, daß mit dem erfindungsgemäßen Testansatz das Fibrinolysesystem inklusive der Komponenten sc-u-PA, sc-t-PA erfaßt wird: erhöhte Plasmaspiegel an sc-u-PA oder freiem sc-t-PA gehen einher mit erhöhter fibrinolytischer Aktivität. Bei zwei der drei Patienten mit anamnestischer Blutungsneigung zeigt sich ein pathologisch erhöhter Ausfall des Fibrinolysetest.

### Beispiel 5

### Fibrinolysetest durch Einsatz von Chelatbildner und Chloramin zur Erfassung von exogenem Plasminogen-Aktivator (hauptsächlich vom Gewebetyp)

100»l humanes Citratplasma a) - e) siehe Beispiel 4 wurden gemäß Beispiel 4 inkubiert mit dem Unterschied, daß Reagenz I durch 25 mM EDTA, 10 mM Chloramin T, 150 mM Tris (Trispuffer konnte auch durch BICIN-Puffer ersetzt werden ), 50 mM NaCl, 0.02 % ^{R}Triton X 100, pH 8.4, ersetzt wurde.

**Tabelle 5**

| Plasma | erzeugte Plasminaktivität (E x 1000) |
|---|---|
| a) Normalplasma | 134 ± 6 |
| b) A2-AP-Mangelplasma | 523 ± 2 |
| c) a) + 10 ng/ml sc-u-PA | 118 ± 1 |
| d) a) + 20 ng/ml sc-u-PA | 114 ± 1 |
| e) a) + 40 IE/ml sc-t-PA | 167 ± 4 |
| f) a) + 80 IE/ml sc-t-PA | 227 ± 5 |

Das Ergebnis zeigt, daß diese Variation eines Fibrinolysetests unabhängig vom sc-u-PA-Gehalt (intrinsic system der Fibrinolyse) ist, jedoch auf unterschiedliche Mengen an (extrinsic) Gewebeplasminogenaktivator gut anspricht.

## Patentansprüche

1. Verfahren zur globalen Erfassung der Komponenten des fibrinolytischen Systems in Plasma oder einer anderen biologischen Flüssigkeit dadurch gekennzeichnet, daß man Plasma oder dieser Flüssigkeit einen Plasminogenaktivator, eine ω̅-Aminokarbonsäure und/oder ein Methionin zu Methioninsulfoxid oxidierendes Agens zusetzt und die entstehende Plasminogenaktivität bestimmt, wonach durch Vergleich mit der Plasminogenaktivität von Normalplasma die globale Funktion der Komponenten des Fibrinolysesystems in Plasma festgestellt wird.

2. Verfahren nach Anspruch 1 dadurch gekennzeichnet, daß als Plasminogenaktivator Urokinase verwendet wird.

3. Verfahren nach Anspruch 1 dadurch gekennzeichnet, daß als Plasminogenaktivator Gewebe-Plasminogenaktivator verwendet wird.

4. Verfahren nach Anspruch 1 dadurch gekennzeichnet, daß der Plasminogenaktivator im Testgemisch erzeugt wird.

5. Verfahren nach Anspruch 4 dadurch gekennzeichnet, daß als plasminogenaktivatoraktivierende Substanz ein Oberflächenaktivator, vorzugsweise Ellagsäure oder ein Sulfatid verwendet wird.

6. Verfahren nach Anspruch 1 dadurch gekennzeichnet, daß das Methionin zu Methionsulfoxid oxidierendes Agens, ein N-Chloramin oder eine andere singlet-Sauerstofffreisetzende Substanz ist.

7. Verfahren nach Anspruch 1 dadurch gekennzeichnet, daß die ω-Aminocarbonsäure Transexamsäure vorzugsweise in einer Konzentration von 0.5 bis 5 mM ist.

8. Verfahren nach Anspruch 3 dadurch gekennzeichnet, daß die Bestimmung in Anwesenheit von t-PA stimulierenden Substanzen durchgeführt wird.

9. Verfahren nach Anspruch 3 dadurch gekennzeichnet, daß die Bestimmung in Anwesenheit eine Chelatbildners, vorzugsweise EDTA, durchgeführt wird.

10. Verfahren nach Anspruch 3 dadurch gekennzeichnet, daß die Bestimmung in einem Einschrittverfahren durchgeführt wird.

## Claims

1. A method for the global measurement of the components of the fibrinolytic system in plasma or another biological fluid, which comprises a plasminogen activator,a ω-amino carboxylic acid and/or an agent which oxidizes methionine to methionine sulfoxide being added to plasma or this fluid, and the resulting plasminogen activity being determined, after which a comparison with the plasminogen activity of normal plasma is used to establish the global function of the components of the fibrinolysis system in plasma.

2. The method as claimed in claim 1, wherein urokinase is used as plasminogen activator.

3. The method as claimed in claim 1, wherein tissue plasminogen activator is used as plasminogen activator.

4. The method as claimed in claim 1, wherein the plasminogen activator is generated in the test mixture.

5. The method as claimed in claim 4, wherein a surface activator, preferably ellagic acid or a sulfatide, is used as plasminogen activator-activating substance.

6. The method as claimed in claim 1, wherein the agent which oxidizes methionine to methionine sulfoxide is an N-chloramine or another singlet oxygen-liberating substance.

7. The method as claimed in claim 1, wherein the ω-amino carboxylic acid is tranexamic acid, preferably in a concentration of 0.5 to 5 mM.

8. The method as claimed in claim 3, wherein the determination is carried out in the presence of t-PA-stimulating substances.

9. The method as claimed in claim 3, wherein the determination is carried out in the presence of a chelating agent, preferably EDTA.

10. The method as claimed in claim 3, wherein the determination is carried out in a one-step method.

## Revendications

1. Procédé pour la détermination globale des composants du système fibrinolytique dans le plasma ou dans un autre liquide biologique, caractérisé en ce que l'on ajoute au plasma ou à ce liquide un activateur du plasminogène, un acide ω-aminocarboxylique et/ou un agent oxydant la méthionine en méthionine-sulfoxyde, et on détermine l'activité plasminogène résultante, à la suite de quoi on détermine, par comparaison avec l'activité plasminogène du plasma normal, la fonction globale des composants du système fibrinolytique dans le plasma.

2. Procédé selon la revendication 1, caractérisé en ce que, comme activateur du plasminogène, on utilise l'urokinase.

3. Procédé selon la revendication 1, caractérisé en ce que, comme activateur du plasminogène, on utiliser activateur tissulaire du plasminogène.

4. Procédé selon la revendication 1, caractérisé en ce que l'activateur du plasminogène est formé dans le mélange d'essai.

5. Procédé selon la revendication 4, caractérisé en ce que, comme substance activant l'activateur du plasminogène, on utilise un activateur de surface, de préférence l'acide ellagique ou un sulfatide.

6. Procédé selon la revendication 1, caractérisé en ce que l'agent oxydant la méthionine en méthionine-sulfoxyde est une N-chloramine ou une autre substance libérant de l'oxygène à singulet.

7. Procédé selon la revendication 1, caractérisé en ce que l'acide ω-aminocarboxylique est l'acide tranexamique, de préférence à une concentration de 0,5 à 5 mM.

8. Procédé selon la revendication 3, caractérisé en ce que la détermination est effectuée en présence de substances stimulant le t-PA.

9. Procédé selon la revendication 3, caractérisé en ce que la détermination est effectuée en présence d'un agent chélateur, l'EDTA de préférence.

10. Procédé selon la revendication 3, caractérisé en ce que la détermination est effectuée dans un procédé en une seule étape.
